# EUROPEAN PATENT SPECIFICATION

(11) **EP 2 452 689 B1**
(45) Date of publication and mention of the grant of the patent: **23.07.2014**
(21) Application number: 11008646.9
(22) Date of filing: 28.10.2011
(51) Int. Cl.: A61K 36/288, A61K 36/53, A61K 36/534, A61P 15/08

(54) **Phythotherapeutic preparation with stimulatory effect on libido and spermatogenesis**
Phytotherapeutische Zubereitung mit stimulierender Wirkung auf Libido, Spermatogenese
Composition phytotherapeutique avec action stimulatoire sur la libido et la spermatogénèse

(30) Priority: 29.10.2010 NL 1038343
(43) Date of publication of application: 16.05.2012
(73) Proprietor: Bozo B.V., 3074 KD Rotterdam (NL)
(72) Inventor: Bujic, Bozo, 3074 KD Rotterdam (NL)
(74) Representative: Klooster, Jan Hanri

(56) References cited:
- EP-A1- 2 184 069
- WO-A1-2005/063266
- WO-A1-2010/066852
- DE-A1- 1 692 045
- FR-A1- 2 914 552
- US-A1- 2008 069 906
- LUBNA H. TAHTAMOUNI ET AL: "Dandelion (Taraxacum officinale) decreases male rat fertility in vivo", JOURNAL OF ETHNOPHARMACOLOGY, vol. 135, no. 1, 1 April 2011 (2011-04-01) , pages 102-109, XP055021592, ISSN: 0378-8741, DOI: 10.1016/j.jep.2011.02.027
- DATABASE WPI Week 200506 Thomson Scientific, London, GB; AN 2005-053145 XP002671731, & JP 2005 002057 A (ICHIMARU PHARCOS INC) 6 January 2005 (2005-01-06)
- S.A. Oran. D.M. Eisawi: "Checklist of Medicinal Plants in Jordan", Medicinal and Biological Sciences, vol. 25, no. 1 1 January 1998 (1998-01-01), XP002672190, University of Jordan Retrieved from the Internet: URL:http://www.ju.edu.jo/sites/Academic/s. oran/Lists/Published%20Research/DispForm.a spx?ID=10 [retrieved on 2012-03-15]

## Description

### Field of the invention.

The invention relates to a phytotherapeutic preparation with a stimulatory effect on the libido and/or spermatogenesis of the human male, and for promoting hygiene of the human genital organs.

More particularly the invention relates to a vegetable preparation, substance or composition that contains as active ingredients only plants, parts of plants or plant materials or combinations thereof for use in a therapy for subjects having symptoms in the area of libido, spermatogenesis and hygiene of the genitals. The invention also relates to a preparation or composition comprising a formulation comprising an extract of plant material of at least one plant species from the family Geraniaceae and/or of at least one plant species from the family Asteraceae, and optionally, a plant species from the family Lamiaceae.

Examples of such plants from the family Geraniaceae are:
G. robertianum, G. Macrorrhizum and plants from the genus Pelargonium.

Examples of plants from the Lamiaceae family are: Satureja hortensis L., Satureja montana L. (savory), Melissa officinalis (lemon balm), Thymus vulgaris (thyme), plants from the genus Mentha as Mentha arvensis (field mint).

Examples of plants from the Asteraceae family are species from the genus Taraxacum like Taraxacum officinale A. (dandelion), from the genus Cichorium (chicory) such as Cichorium intybus (wild chicory) and from the genus Bellis such as Bellis perennis (daisy).

The invention also relates to the use of the preparation or composition in manufacturing an orally active tincture for humans and to the application of the preparation or the tincture in a food supplement and to the application of the food supplement in a food product.

### Background of the Invention

From Serbian patent YU49192 B of the applicant and inventor Obrenović a phytotherapeutic preparation and tincture for internal use is known derived from leaves of the plants G. Robertianum, Satureja hortensis L., Thymus vulgaris, and of plants of the genus Mentha. The preparation may be used as a phytotherapeutic remedy for symptoms indicating disorders within the domain of libido and spermatogenesis, in particular of male humans.

In order to achieve fertilization at sexually reproducing animals, like humans, sperm or ejaculate containing spermatozoa from the male must be brought into contact with an ovum of a female.

For a good chance of a successful fertilization, a number of parameters of the ejaculate are important:
the amount of semen or seminal fluid and its acidity,
sperm counts, both total and per millilitre,
the quality of the spermatozoa, their shape, motility rate and vitality.

For a successful natural fertilization it is also important that during the sexual intercourse the penis of the male inserts the sperm into the interior of the vagina of the female since the lifetime of sperm outside the body is only about 5 minutes.

Libido or sex drive comprises psychological factors such as degree of desire for sexual activities but also, in particular with males, physical aspects and disorders such as erectile dysfunction, whereby the ability to develop and/or maintain a penile erection is insufficient to achieve a successful intercourse and fertilization.

The tincture as disclosed in patent YU49192 is supportive in therapies aiming at increasing the potency, the sexual reflexes and the libido of the male, but also in therapies that promote spermatogenesis, in other words the production and quality of the spermatozoa.

In the patent tests are described, carried out on thirty healthy male volunteers aged from 30 to 35 years, from which is concluded that oral administration of the tincture results In an increase of the sperm count and of the sperm motility rate.

The tinctures of YU49192 are preferably obtained by maceration. The macerate obtained is a "cold" extract of plant material in water at 15 to 25 degrees Celsius. Maceration in water is mainly used for herbs or plants that lose their activity when heated. This process is also used with plants that contain substances that are converted into irritating substances when heated or cooked.

It is also possible to produce a macerate using glycerine, (ethyl)alcohol, oil or other solvents. The physiologically active ingredients can also be extracted from plants by means of ultrasonic extraction.

Usually alcohol is proposed as a solvent for the tincture. In YU49192 a mixture of ethanol (ethyl-alcohol), glycerine and (distilled) water is proposed for the solvent.

Although with the original tincture good results can be achieved, there remains a need for a strengthened and enhanced performance. Also a small number of users has objected to the smell and/or the taste of the tincture and the food supplement, causing a decreased effect of the tincture due to irregular intake.

### Summary of the invention

It is the object of the Invention to provide a phytotherapeutic preparation with an improved and enhanced stimulatory effect on spermatogenesis and libido of the the human male and also with a beneficial effect on body hygiene.

This object is achieved by the invention in providing a preparation according to the preamble that furthermore comprises:
- an extract of the plant Taraxacum officinale Asteraceae, and/or
- an extract of the plant Geraniaceae Melissa officinalis.

Surprisingly it has been found that by oral administration of a preparation containing the abovementioned extract(s) according to the invention a stimulatory effect on the libido and the spermatogenesis of the male is obtained and that furthermore a beneficial effect is obtained on personal hygiene in particular of the genitals, while the taste and odour perception of the preparation is assessed as pleasant.

Preferably the preparation comprises at least an extract of one or more plants selected from the group::
- G. Robertianum
- Satureja hortensis L.
- Thymus L.
- Mentha L.

It appears that by adding one or more of the abovementioned plant extracts a highly efficacious remedy is obtained, having a strong fortified effect while exhibiting an odour with a pleasant character. Adding the extract of Taraxacum officinale A and/or G. Melissa officinalis has a significant synergistic effect on the active ingredients listed in YU49192 bringing about an increased libido and a spermatogenesis-enhancing effect and moreover providing the advantageous property of an agreeable odour, which harmonizes well with the aromas of the other components resulting in a preparation having a complex scent with a pleasant character that the user assesses as positive.

Preferably preparation comprises extract in quantities selected from the ranges:

| | | |
|---|---|---|
| - G. Robertianum | from 40 to | 100 gram |
| - Satureja hortensis L. | from 30 to | 90 gram |
| - Thymus L, | from 40 to | 80 gram |
| - Mentha L. | from 15 to | 50 gram |
| - Melissa Officinalis G. | from 1 to | 10 gram |
| - Taraxacum officinale A. | from 1 to | 5 gram. |

The abovementioned amounts and ratios provide for an excellent active preparation with synergistic effects of the active ingredients.

The embodiment is preferred wherein one or more plant extracts are obtained by maceration or cold extraction. This prevents that the compounds lose their effectiveness when heated. This method also prevents irritating substances being formed out of certain ingredients when heated or cooked.

Preferred is the embodiment of a phytotherapeutic tincture comprising a preparation according to the invention wherein one or more plant extracts are obtained by maceration or cold extraction. In this form, the preparation can be administered orally and the body can take up the active substances easily.

In a preferred embodiment the invention is characterized in that the tincture comprises solvent in quantities selected from the ranges:

| | | |
|---|---|---|
| - Glycerine | from 70 to | 150 gram |
| - Ethyl-alcohol | from 300 to | 450 gram |
| - Water | from 500 to | 700 gram |

The abovementioned amounts and ratio's provide for an excellent active tincture, which can be taken orally, and which gives rise to synergistic effects between the active components.

Advantageous is the embodiment wherein tincture has the following approximate composition in percent by weight:

| | | |
|---|---|---|
| - G. Robertianum | 5% | ± 5% |
| - Satureja hortensis L. | 5% | ± 5% |
| - Thymus L. | 5% | ± 5% |
| - Mentha L. | 1,5% | ± 1,5% |
| - Melissa Officinaiis G. | 5% | ± 5% |
| - Taraxacum officinale A. | 2,5% | ± 2,5% |
| - Glycerine | 7% | ± 5% |
| - Ethyl-alcohol | 25% | ± 10% |
| - Water | 43% | ± 20% |

A tincture of plant extracts having the abovementioned ratios exhibits a synergistic effect on libido and spermatogenesis of the human male, helps with the hygiene of the genital organs and has an olfactory perception, which is perceived as pleasant

Preferably an embodiment of a food supplement comprises a phytotherapeutic preparation according to the invention or a phytotherapeutic tincture according to the invention.

Preferred is the embodiment of a food product that comprises a food supplement including a preparation and/or tincture according to the invention.

Description of the method of production of the preparations and tinctures.

The extracts of the plants are preferably prepared by means of maceration at room temperature. The prescribed amounts of finely comminuted leaves of the plants are extracted in a solvent such as water, during 10 - 60 days protected from light. Preferably each day the mixture of leaves and solvent is agitated and/or stirred. This is then followed by separating the solid from the liquid by means of sieves. By hand or mechanically the maceration mixture is pressed or squeezed through one or more sieves provided with different mesh sizes.

As a result an extraction efficiency of about 60-85% is obtained based on the tincture solvent The tincture must then be left alone for 2-6 days to allow the suspended particles to settle. In a next step the precipitate is decanted and the tincture is subsequently filtered on double gauze and cotton wool after which the tincture is stored in brown glass bottles with a capacity of 100 - 200 grams or cc (cl).

The extracts can also be prepared by means of ultrasound extraction in an ultrasonic bath using ultrasonic vibrations at a predetermined volume, by pouring solvent over the required amounts of plant leaves in the requisite degree of comminution.

During a period of 25-40 minutes, the extraction is performed as the comminuted plant leaves absorb the ultrasonic waves, while simultaneously the mixture is heated to 45 degrees C.

After extraction, the tincture is further treated and purified by sieving the mixture on several sieves having different mesh sizes and then manually or mechanically pressing or squeezing the mixture through one or more sieves.

Thereby an extraction efficiency of about 60-85% is obtained based on the tincture solvent. The tincture must then for 4-6 days be allowed to let the suspended particles settle and precipitate. This is then followed by decanting and subsequently filtering the tincture using a double filter with cotton and thereafter storing the tincture in brown glass bottles with a capacity of 100 - 200 grams or cc (cl).

Use is made of plants having a standardized quality and of solvents according to the formulation. The quality of products ensures that active components and substances are readily dissolved in the preparation or in the tincture.

| | Recipe. | |
|---|---|---|
| - G. Robertianum | from 40 to | 100 gram |
| - Satureja hortensis L. | from 30 to | 90 gram |
| - Thymus L. | from 40 to | 80 gram |
| - Mentha L. | from 15 to | 50 gram |
| - Melissa Officinalis G. | from 1 to | 10 gram |
| - Taraxacum officinale A. | from 1 to | 5 gram |
| - Glycerine | from 70 to | 150 gram |
| - Ethyl-alcohol | from 300 to | 450 gram |
| - Water | from 500 to | 700 gram |

A chemical analysis performed by a laboratory has revealed that the plant extracts and tincture among others include: essential oil, tannins (corilagine, a gallotannin) and phenols (such as thymol, carvacrol, cinnamic acid or cinnamate ester), and flavonoids, phenolic heteroglucosides (phenolic glycosides) or arbutin and bitter compounds, resulting in mutual synergy.

Laboratory tests of the tincture provided the following ranges for the active ingredients in 100 g tincture in weight percent:

| | | |
|---|---|---|
| - G. Robertianum | 5% | ± 5% |
| - Satureja hortensis L. | 5% | ± 5% |
| - Thymus L. | 5% | ± 5% |
| - Mentha L. | 1,5% | ± 1,5% |
| - Melissa Officinalis G. | 5% | ± 5% |
| - Taraxacum officinale A. | 2,5% | ± 2,5% |
| - Glycerine | 7% | ± 5% |
| - Ethyl-alcohol | 25% | ± 10% |
| - Water | 43% | ± 20% |

Tests with the tincture in Serbia on thirty healthy male volunteers, aged 30-35 years, indicated an increase in the amount of the sperm (sperm count) ranging from 2-189% and an increase in motility of the sperm of 4-385%, as a result of a daily intake of 30 gram tincture.

## Claims

1. Phytotherapeutic preparation comprising extracts from the plants:
- G. Robertianum, and
- Satureja hortensis L., and
- Thymus L., and
- Mentha L.
**characterized in that** the preparation at least comprises an extract from:
- Taraxacum officinale Asteraceae, and/or
- Melissa officinalis Geraniaceae

2. Phytotherapeutic preparation according to claim 1, wherein the preparation comprises extracts in quantities selected from the ranges:
| | | |
|---|---|---|
| - G. Robertianum | from 40 to | 100 gram |
| - Satureja hortensis L. | from 30 to | 90 gram |
| - Thymus L. | from 40 to | 80 gram |
| - Mentha L. | from 15 to | 50 gram |
| - Melissa Officinalis G. | from 1 to | 10 gram |
| - Taraxacum officinale A. | from 1 to | 5 gram |

3. Phytotherapeutic preparation according to any one of the claims 1 - 2, wherein one or more plant extracts are obtained by maceration or cold extraction.

4. Phytotherapeutic tincture comprising a preparation according to any one of the claims 1 - 3, wherein a mixture of glycerine, ethyl-alcohol and water is used as solvent.

5. Phytotherapeutic tincture according to claim 4, wherein the tincture comprises solvent in quantities selected from the ranges:
| | | |
|---|---|---|
| - Glycerine | from 70 to | 150 gram |
| - Ethyl-alcohol | from 300 to | 450 gram |
| - Water | from 500 to | 700 gram |

6. Phytotherapeutic tincture according to claim 5, wherein the tincture has the following approximate composition in percent by weight:
| | | |
|---|---|---|
| - G. Robertianum | 10% or less | |
| - Satureja hortensis L. | 10% or less | |
| - Thymus L. | 10% or less | |
| - Mentha L. | 3% or less | |
| - Melissa Officinalis G. | 10% or less | |
| - Taraxacum officinale A. | 5% or less | |
| - Glycerine | 7% | ±5% |
| - Ethyl-alcohol | 25% | ±10% |
| - Water | 43% | ±20% |

7. Food supplement comprising a phytotherapeutic preparation according to any one of the claims 1 - 3 or a phytotherapeutic tincture according to any one of the claims 5-6.

8. Food product comprising a food supplement according to claim 7.

## Patentansprüche

1. Pflanzentherapeutisches Präparat wobei das Präparat Pflanzenextrakten umfasst aus:
- G. Robertianum, und
- Satureja hortensis L., und
- Thymus L., und
- Mentha L.
**dadurch gekennzeichnet, dass** das Präparat mindestens ein Extrakt umfasst aus:
- der Asteraceae-Pflanze Taraxacum officinale und/oder
- der Geraniaceae-Pflanze Melissa officinalis.

2. Pflanzentherapeutisches Präparat nach Anspruch 1, wobei das Präparat Pflanzenextrakten in mengen umfasst, die aus folgenden Bereichen ausgewählt sind:
| | | |
|---|---|---|
| - G. Robertianum | von 40 bis | 100 Gramm |
| - Satureja hortensis L. | von 30 bis | 90 Gramm |
| - Thymus L. | von 40 bis | 80 Gramm |
| - Mentha L. | von 15 bis | 50 Gramm |
| - Melissa Officinalis G. | von 1 bis | 10 Gramm |
| - Taraxacum officinale A. | von 1 bis | 5 Gramm |

3. Pflanzentherapeutisches Präparat nach einem der Ansprüche 1-2, wobei ein oder mehrere Pflanzenextrakte durch Mazeration oder Kälteextraktion erhalten werden.

4. Pflanzentherapeutische Tinktur, die ein Präparat nach einem der Ansprüche 1-3 umfasst, wobei ein Gemisch von Glycerin, Ethylalkohol und Wasser als Lösemittel verwendet wird.

5. Pflanzentherapeutische Tinktur nach Anspruch 4, wobei die Tinktur Lösemittel in Mengen umfasst, die aus folgenden Bereichen ausgewählt sind:
| | | |
|---|---|---|
| - Glycerin | von 70 bis | 150 Gramm |
| - Ethylalkohol | von 300 bis | 450 Gramm |
| - Wasser | von 500 bis | 700 Gramm. |

6. Pflanzentherapeutische Tinktur nach Anspruch 5, wobei die Tinktur die folgende ungefähre Zusammensetzung in Gewichtsprozent aufweist:
| | | |
|---|---|---|
| - G. Robertianum | 10% | oder weniger |
| - Satureja hortensis L. | 10% | oder weniger |
| - Thymus L. | 10% | oder weniger |
| - Mentha L. | 3% | oder weniger |
| - Melissa Officinalis G. | 10% | oder weniger |
| - Taraxacum officinale A. | 5% | oder weniger |
| - Glycerin | 7% | ± 5% |
| - Ethylalkohol | 25% | ± 10% |
| - Wasser | 43% | ± 20%. |

7. Nahrungsergänzungsmittel, das ein pflanzentherapeutisches Präparat nach einem der Ansprüche 1-3 oder eine pflanzentherapeutische Tinktur nach einem der Ansprüche 5-6 umfasst.

8. Nahrungsmittelprodukt, das ein Nahrungsergänzungsmittel nach Anspruch 7 umfasst.

## Revendications

1. Préparation phytothérapeutique comprenant des extraits végétaux de :
- G. Robertianum, et
- Satureja hortensis L., et
- Thymus L., et
- Mentha L.
**caractérisée en ce que** la préparation comprend au moins un extrait de :
- la plante Taraxacum officinale Asteraceae, et/ou
- la plante Melissa officinalis Geraniaceae.

2. Préparation phytothérapeutique selon la revendication 1, dans laquelle la préparation comprend des extraits dans des quantités choisies dans les portions suivantes :
| | | |
|---|---|---|
| - G. Robertianum | de 40 à | 100 grammes |
| - Satureja hortensis L. | de 30 à | 90 grammes |
| - Thymus L. | de 40 à | 80 grammes |
| - Mentha L. | de 15 à | 50 grammes |
| - Melissa Officinalis G. | de 1 à | 10 grammes |
| - Taraxacum officinale A. | de 1 à | 5 grammes |

3. Préparation phytothérapeutique selon l'une quelconque des revendications 1 à 2, dans laquelle un ou plusieurs extraits végétaux sont obtenus par macération ou extraction à froid.

4. Teinture phytothérapeutique comprenant une préparation selon l'une quelconque des revendications 1 à 3, dans laquelle un mélange de glycérine, d'éthanol et d'eau est utilisé comme solvant.

5. Teinture phytothérapeutique selon la revendication 4, dans laquelle la teinture comprend le solvant dans des quantités choisies dans les proportions suivantes :
| | | |
|---|---|---|
| - Glycérine | de 70 à | 150 grammes |
| - Ethanol | de 300 à | 450 grammes |
| - Eau | de 500 à | 700 grammes |

6. Teinture phytothérapeutique selon la revendication 5, dans laquelle la teinture à la composition approximative suivante en pourcentages pondéraux :
| | | |
|---|---|---|
| - G. Robertianum | 10 % | ou moins |
| - Satureja hortensis L. | 10 % | ou moins |
| - Thymus L. | 10 % | ou moins |
| - Mentha L. | 3% | ou moins |
| - Melissa Officinalis G. | 10 % | ou moins |
| - Taraxacum officinale A. | 5% | ou moins |
| - Glycérine | 7 % | ±5 % |
| - Ethanol | 25 % | ±10 % |
| - Eau | 4 3% | ± 20 % |

7. Complément alimentaire comprenant une préparation phytothérapeutique selon l'une quelconque des revendications 1 à 3 ou une teinture phytothérapeutique selon l'une quelconque des revendications 5 et 6.

8. Produit alimentaire comprenant un complément alimentaire selon la revendication 7.
